(19) Europäisches Patentamt
European Patent Office
Office européen des brevets

(11) **EP 2 767 230 B1**

(12) **EUROPEAN PATENT SPECIFICATION**

(45) Date of publication and mention
of the grant of the patent:
**13.05.2020 Bulletin 2020/20**

(51) Int Cl.:
*A61B 5/04* (2006.01)  *A61B 5/00* (2006.01)
*G01R 35/00* (2006.01)  *G01R 27/14* (2006.01)

(21) Application number: **14155380.0**

(22) Date of filing: **17.02.2014**

(54) **System for impedance measurements**

System für Impedanzmessungen

Système de mesure d'impédance

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.02.2013 EP 13155550**

(43) Date of publication of application:
**20.08.2014 Bulletin 2014/34**

(73) Proprietor: **IMEC VZW**
**3001 Leuven (BE)**

(72) Inventors:
• **Yazicioglu, Refet Firat**
**3001 Leuven (BE)**

• **Kim, Sunyoung**
**3001 Leuven (BE)**

(56) References cited:
**EP-A2- 2 298 164   US-A- 5 467 090**

• **YAN LONG ET AL: "A 13 muA Analog Signal Processing IC for Accurate Recognition of Multiple Intra-Cardiac Signals", IEEE TRANSACTIONS ON BIOMEDICAL CIRCUITS AND SYSTEMS, IEEE, US, vol. 7, no. 6, 20 January 2014 (2014-01-20), pages 785-795, XP011538076, ISSN: 1932-4545, DOI: 10.1109/TBCAS.2013.2297353 [retrieved on 2014-01-24]**

# Description

## Technical Field

The present disclosure relates generally to systems and devices for performing measurement of physical qualities based on a change of an electrical impedance, and more specifically to impedance measurement systems.

## Background

Conventional impedance measurement systems and circuits are generally based on injecting a differential current signal into the unknown source of impedance and measuring the resulting differential voltage. Biomedical application systems often require high precision impedance measurements, for example down to milliohm range to detect variable impedance changes superimposed on up to kilo-ohm range baseline impedances. The limitations of integrated circuit design, due to the effect of non-linearity, mismatch, and offset, lead to errors and reduce the accuracy of such impedance measurements. Therefore, there is the need to improve the accuracy and/or reduce error when measuring an electrical impedance.

A known biomedical acquisition system that performs impedance monitoring using two-terminals, also known as a bipolar electrode configuration, is disclosed in patent application EP 2 298 164. The impedance measurement circuit, as in the exemplary embodiment shown in figure 27 of that document, comprises two AC stimulation current sources that generate a differential mode stimulation current through two electrodes. Additionally, an impedance readout circuit in the analogue domain is configured to extract, from the analogue signals received from those two electrodes, the imaginary and real parts of the measured electrode-tissue impedance.

Another known biomedical acquisition system that performs AC impedance measurements is disclosed in US patent 5,467,090. The circuit comprises a current generator unit configured for generating a differential alternate current signal that is injected to a patient that presents a certain electrical impedance: electrode sensors for providing an analogue sensor signal sensed from that patient: a filtering unit for adapting said analogue sensor signal: a calibration circuit comprising a switch matrix connected to a chopper: an analogue impedance readout unit for calculating an impedance value signal: an ADC unit for digitizing the impedance signal generated by the analogue impedance readout unit; and a DSP unit configured to receive a digital version of the impedance signal and to store it in a memory unit.

## Summary

According to one aspect of the present invention, a new system with improved impedance measurement is provided. According to another aspects, the invention is also advantageous for use in ambulatory and/or low power biomedical monitoring applications. The invention is particularly advantageous for the measurements of bio-impedance information. Other possible applications are, for example, galvanic skin response or skin impedance measurement, impedance cardiography or contact impedance.

According to an exemplary embodiment, there is provided a system for impedance measurement according to claim 1.

The system comprises an ADC unit configured to digitize the impedance signal generated by the analogue impedance readout unit; and a DSP unit configured to receive a digital version of the impedance signal and to store it in a memory unit; wherein, in operation, during the calibration period, the DSP unit is configured to store a value of an offset error related to the analogue impedance readout unit and the ADC unit; and wherein, in operation, during the measurement period, the DSP unit is configured to calculate an electrical impedance value by subtracting the offset error from the digitized impedance value signal calculated at the analogue impedance readout unit.

The system comprises a current generator unit configured to generate a differential alternate current signal to an element that presents a certain electrical impedance.

According to an exemplary embodiment, during the calibration period, the circuit is configured to activate the chopper by providing a frequency signal which has the same frequency as the differential alternate current signal generated by the current generator unit.

The filtering unit is a high-pass filter unit configured to adapt the received analogue sensor signal so as to reject DC offset.

According to an exemplary embodiment, the calculated offset error comprises a DC offset and drift due to process, voltage and temperature variations of the analogue impedance readout unit.

According to an exemplary embodiment, the impedance measurement is a bio-impedance measurement.

There is also provided a biopotential signal acquisition system and an electronic device comprising a system for impedance measurements according to any of the embodiments herein described.

Advantageously, by using the circuit and system according to exemplary embodiments of the invention, the offset error in the analogue readout channel is advantageously reduced. The offset error may include process, temperature and/or voltage variations. According to exemplary embodiments, in operation, an calculation error correction is performed, first measuring the error in the analogue readout channel and then subtracting this error from the impedance measurements. Advantageously, impedance measurement accuracy is increased. According to exemplary embodiments, the solution improves the accuracy of a DC bio-impedance measurement.

Certain objects and advantages of various new and inventive aspects have been described above. It is to be understood that not necessarily all such objects or advantages may be achieved in accordance with any particular embodiment of the present invention. Those skilled in the art will recognize that the solution of the present invention may be embodied or carried out in a manner that achieves or optimizes one advantage or group of advantages without necessarily achieving other objects or advantages.

## Brief description of the drawings

The above and other aspects of the circuit, system and method for impedance measurement according to the present invention will be shown and explained with reference to the non-restrictive example embodiments described herei nafter.

Figure 1 shows a first exemplary block diagram of a system for impedance measurements according to an embodiment of the invention.
Figure 2 shows a second exemplary block diagram of a system for impedance measurements according to an embodiment of the invention.
Figure 3 shows a first exemplary block diagram of a circuit for impedance measurements according to an embodiment of the invention.
Figure 4 shows a second exemplary block diagram of a circuit for impedance measurements according to an embodiment of the invention.
Figures 5A and 5B show a third exemplary block diagram of a circuit for impedance measurements in operation according to an embodiment of the invention.
Figures 6A and 6B show a fourth exemplary block diagram of a circuit for impedance measurements in operation according to an embodiment of the invention.
Figures 7A and 7B show a fifth exemplary block diagram of a circuit for impedance measurements in operation according to an embodiment of the invention.
Figure 8 illustrates a flow diagram of a method for impedance measurements.
Figure 9 illustrates a third exemplary block diagram of a system for impedance measurements according to an embodiment of the invention.

## Detailed description

In the following, in the description of exemplary embodiments, various features may be grouped together in a single embodiment, figure, or description thereof for the purpose of streamlining the disclosure and aiding in the understanding of one or more of the various inventive aspects. This is however not to be interpreted as the invention requiring more features than the ones expressly recited in the main claim. Furthermore, combinations of features of different embodiments are meant to be within the scope of the invention, as would be clearly understood by those skilled in the art. Additionally, in other instances, well-known methods, structures and techniques have not been shown in detail in order not to obscure the conciseness of the description.

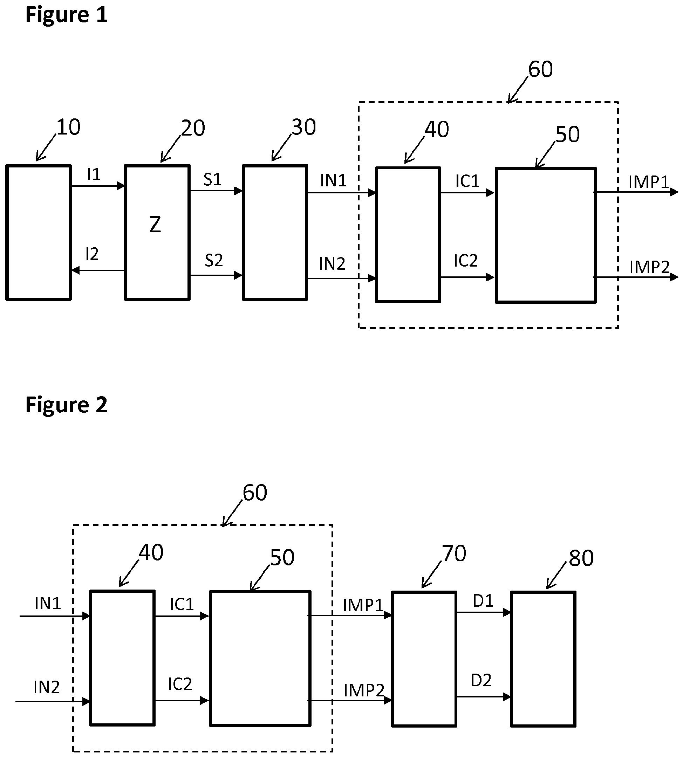

**Figure 1** shows a first exemplary block diagram of a system for impedance measurements according to an embodiment of the invention. Such a system may be, for example, a biopotential signal acquisition system, in connection with the acquisition of biopotential signals such as electrocardiogram (ECG), electroencephalogram (EEG) or electromyogram (EMG) signals. The system may comprise a current generator unit **10,** a high-pass filter (HPF) unit **30,** a calibration unit **40** and an impedance readout unit 50. The calibration unit **40** and an impedance readout unit **50** are implemented, according to an embodiment of the invention, in an analogue domain part **60** of the system. The current generator unit **10** is configured to generate a differential alternate current signal **I1**, **I2** to an element **20** that presents a certain electrical impedance **Z,** such as, for example, a living being body. By means of electrode sensors connected to that element **20,** analogue sensor signals **S1**, S2 are generated and passed through a HPF **30,** which, according to an exemplary embodiment, implements a first order filter with a cut-off frequency that rejects large direct current (DC) offset. According to an exemplary embodiment, the cut-off frequency is lower than the frequency of the stimulation current or input voltage. Then, the calibration unit **40** is configured to receive the analogue signals **IN1,IN2** at the output of the HPF unit **30** and to control and modify those signals for impedance measurement calibration purposes. The output analogue signals **IC1, IC2** of the calibration unit **40** are then provided to an analogue impedance readout unit **50,** which is configured to calculate impedance value signals **IMP1, IMP2** of the electrical impedance **Z** associated to the subject element **20.** According to an exemplary embodiment, the analogue impedance readout unit **50** is configured to extract a real and an imaginary impedance value associated to the measured subject's electrical impedance **Z.**

The current generator unit **10,** the high-pass filter (HPF) unit **30** and an impedance readout unit **50** may be implemented as disclosed in patent application EP 2 298 164, but it is understood that there are other design options to implement the functionality of each of those units. The present disclosure focuses on the development of new and inventive calibration unit **40** or circuit for impedance measurement, together with a system and method for impedance measurements which allows compensation of impedance measurement errors introduced by the analogue impedance readout unit **50.**

Additionally, according to an embodiment of the present invention, the system performs impedance measurements employing a tetra-polar electrode configuration, which means that the current source electrodes are physically separated from the sensing voltage electrodes. Advantageously, this configuration provides higher impedance measurement accuracy than a system for impedance measurements with a bipolar electrode configuration.

**Figure 2** shows a second exemplary block diagram of a system for impedance measurements according to an embodiment of the invention. The system may comprise the same units as the ones shown in **Figure 1** and additionally an analogue-to-digital converter (ADC) unit 70 which digitizes the impedance signals **IMP1, IMP2** and provides a digital version D1, D2 to a digital signal processing (DSP) unit **80.** The solution according to an embodiment of the invention is advantageous due to the fact that the value (or an averaged value) of the impedance signals **IMP1, IMP2** can be stored in a memory unit during a calibration phase. Advantageously, by storing the value of the impedance signals **IMP1, IMP2** associated to calibration, the need for continuous recalibration of the system is avoided. According to an exemplary embodiment, such memory unit is comprised in the DSP unit **80.** Furthermore, according to an embodiment of the invention, there is provided a calibration unit **40** and a method for calibration of impedance measurements which allows compensation of the impedance measurement errors introduced by the analogue impedance readout unit **50** and the analogue-to-digital converter (ADC) unit 70.

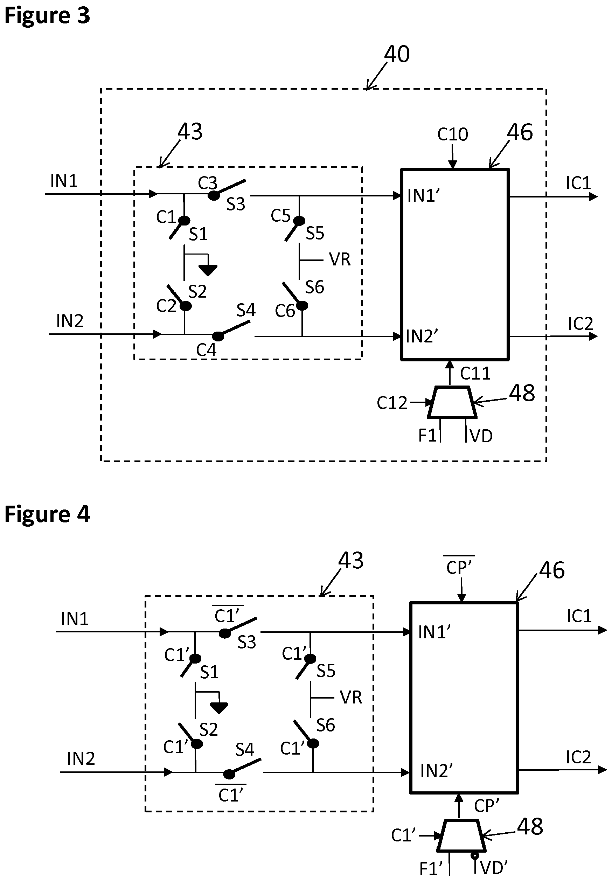

**Figure 3** shows a first simplified block diagram of a calibration unit **40** according to an exemplary embodiment of the invention. Schematically, it comprises a switch matrix **43** connected to a chopper **46.** The switch matrix **43,** according to an embodiment of the invention, comprises six switches **S1 to S6** controlled by a respective switch control signal **C1 to C6.** The chopper **46** is controlled by a multiplexer **48,** which provides a first control signal **C11** depending on the value of a selection signal **C12.** The first control signal **C11** of the chopper **46** may be a frequency signal **F1** for modulation purposes when the chopper is in operation (activated) or a voltage signal **VD** when the chopper is deactivated. The chopper also receives a second control signal **C10** which is related to the value of the first control signal **C11.**

**Figure 4** shows a second exemplary block diagram of a calibration unit **40** according to an embodiment of the invention, which schematically illustrates the relation of the values of the control signals of **Figure 3.** For example, the first, second, fifth and sixth switches **S1, S2, S5, S6** and the multiplexer 48 are controlled by the same first switch control signal C1'. The third and fourth switches S3, S4 are controlled by the inverse value /C1' of the first switch control signal. The chopper is controlled by the first chopper control signal CP' and the inverse value /CP' of that signal.

The chopper **46** may be implemented as disclosed in ″A CMOS chopper amplifier″, by Christian Enz, IEEE Journal of Solid-State Circuits, Vol. SC-22, no 3, June 1987. According to an embodiment of the invention, the frequency signal **F1'** is the demodulation frequency at 0 degrees as used in the analogue impedance readout unit **50** implemented as disclosed in patent application EP 2 298 164.

According to an exemplary embodiment, the frequency signal **F1'** may be the same as the frequency of the current **I1, I2** injected by the current generator unit **10.**

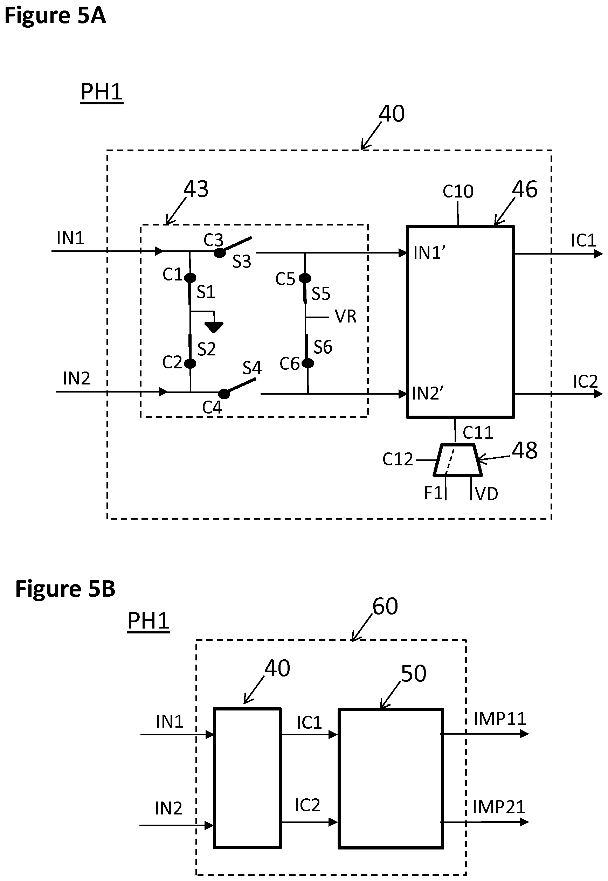

**Figure 5A** shows a third exemplary block diagram of a calibration unit **40,** in operation during a calibration phase **PH1,** according to an embodiment of the invention. During the calibration phase **PH1,** the first, second, fifth and sixth switches **S1, S2, S5, S6** are closed and the third and fourth switches **S3, S4** are opened so that the analogue calibration unit input signals **IN1, IN2** are disconnected from the chopper inputs **IN1', IN2'.** The analogue calibration unit input signals **IN1, IN2** are connected to a ground reference, the inputs of the chopper **46** are connected to a voltage reference signal VR and the chopper is activated by providing the frequency signal F1 as the first control signal C11.

Advantageously, according to an embodiment of the invention, during the calibration phase PH1, the impedance signals **IMP11, IMP21** as shown in **Figure 5B** comprise the offset error of the analogue impedance readout unit 50. The stored offset error may be the combination of the systematic and random offset of the building blocks of the impedance readout unit. According to another exemplary embodiment, the impedance signals **IMP11, IMP21,** during the calibration phase PH1, comprise the value of the DC offset and drift due to process, voltage and temperature variations of the analogue impedance readout unit **50.** According to an exemplary embodiment, and in reference to **Figure 2,** the digital versions **D1, D2** of the impedance signals **IMP11, IMP21** further comprise, during the calibration phase **PH1,** the offset error of the ADC unit **70.** According to an exemplary embodiment, the value (or an averaged value) of the impedance signals **IMP11, IMP21** is, during the calibration phase **PH1,** stored in the memory of the DSP unit **80.**

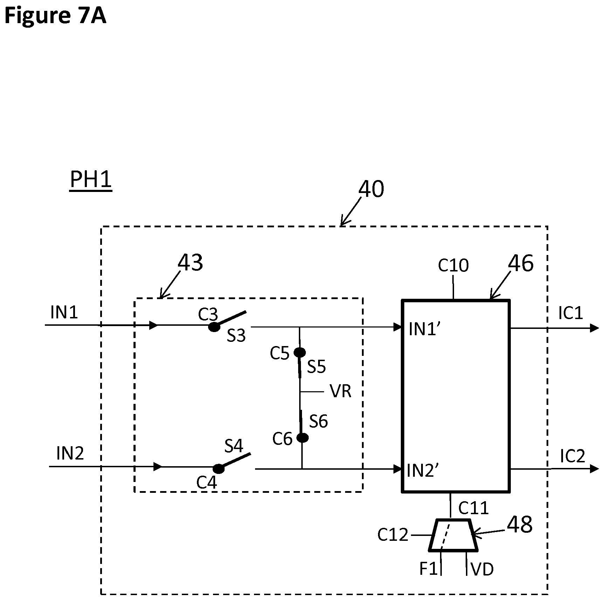

Another possible embodiment of the switch matrix **43,** in operation during a calibration phase **PH1,** is shown in **Figure 7A.** According to an exemplary embodiment, the switch matrix **43** does not comprise the first and the second switches **S1, S2.** During the calibration phase **PH1,** the fifth and sixth switches **S5, S6** are closed and the third and fourth switches **S3, S4** are opened so that the analogue calibration unit input signals **IN1, IN2** are disconnected from the chopper **46** and the chopper inputs **IN1', IN2'** are connected to a voltage reference signal **VR.**

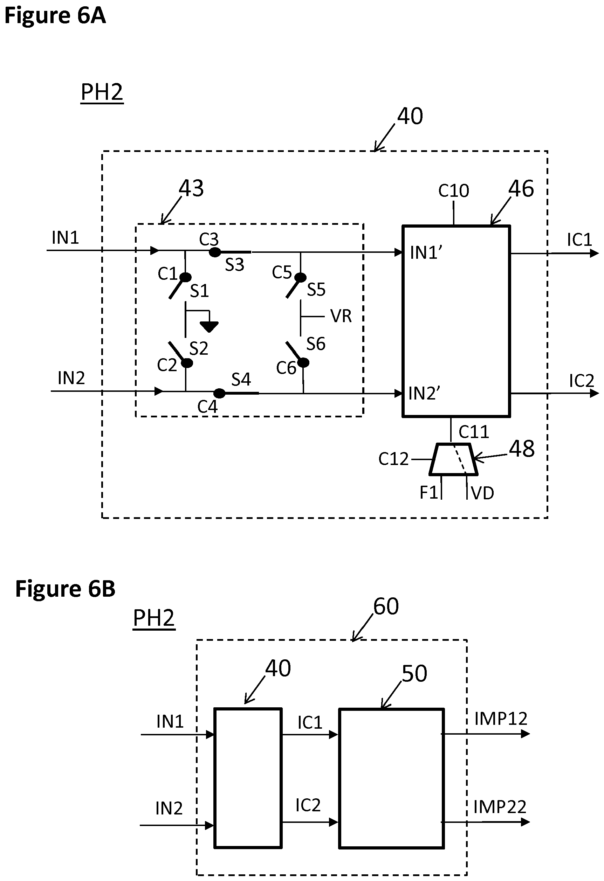

**Figures 6A** shows a fourth exemplary block diagram of a calibration unit **40,** in operation during a measurement phase **PH2,** according to an embodiment of the invention. During the measurement phase **PH2,** the first, second, fifth and sixth switches **S1, S2, S5, S6** are opened and the third and fourth switches **S3, S4** are closed so that the analogue calibration unit input signals **IN1, IN2** are connected to the chopper **46**. The voltage reference signal **VR** is disconnected from the chopper inputs and the chopper is deactivated by providing a voltage signal **VD** as the first control signal **C11**. Since the chopper is deactivated, the calibration unit **40** lets the input signals **IN1, IN2** go through to the analogue impedance readout unit **50,** which then measures the voltage value generated on the impedance **Z** by the injected current **I1, I2**. During the measurement phase **PH2**, the impedance signals **IMP12, IMP22,** as shown in **Figure 6B,** comprise the value of the measured electrical impedance **Z** and the offset error of the analogue impedance readout unit **50**. Furthermore, according to an exemplary embodiment in reference to **Figure 2,** the digital versions **D1, D2** of the impedance signals **IMP12, IMP22** further comprise, during the measurement phase **PH2,** the offset error of the ADC unit **70.**

Advantageously, according to an embodiment of the invention, during the measurement phase PH2, the value of the impedance signals **IMP11, IMP21** obtained during the calibration phase PH1, is subtracted from the value of the impedance signals **IMP12, IMP22** obtained during the measurement phase PH2. Therefore, the system, during the measurement phase PH2, provides a value for the measured electrical impedance Z that does not contain the offset error of the analogue impedance readout unit 50. According to another exemplary embodiment, the system, during the measurement phase **PH2,** provides a value for the measured electrical impedance **Z** that does not contain the offset error of both the analogue impedance readout unit **50** and the ADC unit **70.**

Advantageously, according to an embodiment of the invention, the accuracy of a DC bio-impedance measurement is increased, particularly for baseline impedance measurements.

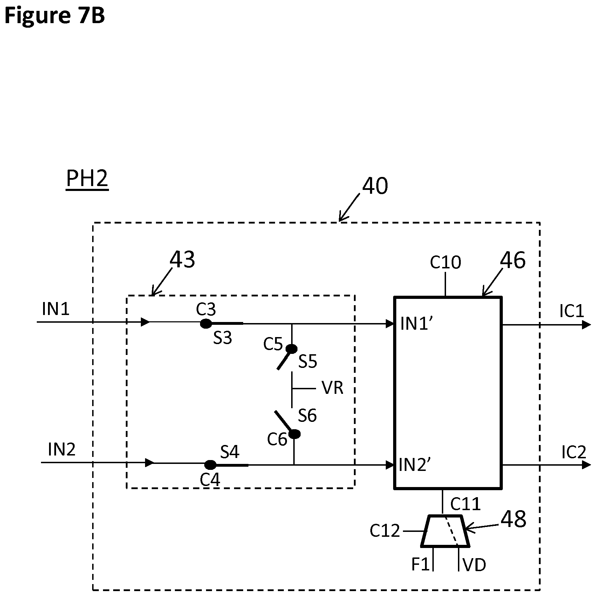

Another possible embodiment of the switch matrix **43,** in operation during a measurement phase **PH2,** is shown in **Figure 7B**. According to an exemplary embodiment, the switch matrix **43** does not comprise the first and the second switches **S1, S2**. During the measurement phase **PH2,** the fifth and sixth switches **S5, S6** are opened and the third and fourth switches **S3, S4** are closed so that the analogue calibration unit input signals **IN1, IN2** are connected to the chopper **46** and the voltage reference signal VR is disconnected from the chopper inputs.

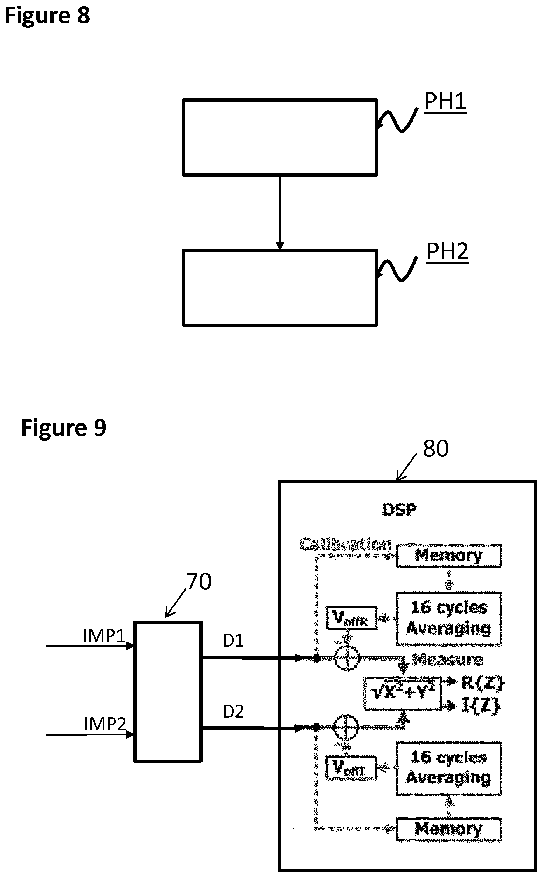

**Figure 8** illustrates a flow diagram of a method for impedance measurements as performed by the system of the invention.

The method comprises, in a first phase or calibration phase **PH1,** calculating the impedance offset error value of an analogue impedance readout unit **50** (as in the system shown in **Figure 1)**. According to an exemplary embodiment, the method further comprises, in a first phase or calibration phase **PH1,** further calculating the offset error of an ADC unit **70**, as in the system shown in **Figure 2**. According to an exemplary embodiment, the method further comprises, in a first phase or calibration phase PH1, storing the calculated offset error in a memory unit. According to an exemplary embodiment, the impedance measurement is a bio-impedance measurement and the offset error of the analogue impedance readout unit **50** or the ADC unit **70** comprises a DC bio-impedance offset error.

The method further comprises, in a second phase or measurement phase **PH2,** measuring the electrical impedance value Z of a subject element **20** (as in the system shown in **Figure 1)** and subtracting from that measured electrical impedance value, the impedance offset error value calculated during first phase or calibration phase **PH1.**

According to an exemplary embodiment, the impedance offset error value and the measured electrical impedance value are voltage values.

According to an exemplary embodiment, the method for impedance measurements according to the invention is implemented in a system (as in **Figure 1** or Figure 2) comprising a calibration unit **40** according to any of the embodiments herein claimed. Additionally, the method for impedance measurements according to the invention may make use of a DSP unit **80.**

The calibration phase and the measurement phase are performed during determined periods of time, which can be selected by design. The calibration phase may be performed once or repeated at specific steps or events of the impedance measurement.

**Figure 9** illustrates another exemplary block diagram of a system for impedance measurements according to an embodiment of the invention, in which the functioning of the DSP unit **80** is further detailed in relation to the system disclosed in **Figures 1 and 2**. During operation of the system, in a calibration phase **PH1,** the voltage values of the calculated impedance offset error **VoffR, VoffI** are stored in a memory. According to an exemplary embodiment, the impedance offset error voltage value is averaged during a certain number of operation cycles. Further during operation of the system, in a measurement phase **PH2,** the stored voltage value of the calculated impedance offset error **VoffR, VoffI** is subtracted from the voltage value of the measured impedance and from that resulting voltage value, the actual value of the measured impedance is calculated **R{Z}, I{Z],**

Further exemplary embodiments are here below described. According to an exemplary embodiment, there is provided a new circuit topology and switch matrix for implementing a new method for impedance measurements. Such solution, according to an exemplary embodiment, advantageously enables to sample the error in the impedance measurement readout so that it can be subtracted from actual impedance measurements.

According to one exemplary embodiment, the solution proposes an error correction mechanisms that first measures the error in the integrated circuits and then subtracts this error from the measurements. According to one exemplary embodiment there is provided a method for impedance measurements comprising: measuring the error of the readout system, sampling the error and subtracting it from the measurement.

According to an exemplary embodiment of the present description, the input switch matrix is first connected to a known potential, which enables to measure the offset of the readout chain. This offset is stored on the DSP for error correction. Later the impedance measurement is started where the measurements are made and error is subtracted from the recordings.

Advantageously, according to an exemplary embodiment of the present description, the error is digitized and stored in memory. Therefore it avoids the need for re-calibration.

According to an exemplary embodiment of the invention, an off-chip HPF will be integrated to interface with the impedance signal path. The impedance signal path comprises an instrumentation amplifier (TC and TI stage), a programmable gain amplifier (PGA), a SC-LPF, and a channel buffer. A test purpose multiplexed buffer (TMPX) may also be integrated to monitor internal node of the channel. Two impedance readout channels are responsible for extracting the in-phase (IMPR) and quadrature (IMPI) components of the bioimpedance.

The HPF may implement a first order filter with a cut-off frequency between 0.1 Hz and 50 Hz to reject large DC offset at input.

The instrumentation amplifier itself consists of a transconductance (TC) stage followed by a transimpedance stage (TI). The instrumentation amplifier (IA) has single ended topology, and it operates inside choppers to improve low frequency noise performance and CMRR. The next building block in the signal path is a programmable-gain amplifier (PGA). This module allows to be configured with different gain settings (6dB-26dB). A reference PGA signal (900mV) is used for the reference node around which the readout path output will be centered. A first order SC-LPF is cascaded after the PGA. This LPF has fixed the BW of IMP signal path to 10Hz. Finally a channel buffer is implemented.

The offset of the quadrature impedance readout circuit may affect the accuracy of the DC bio-impedance calculation. To improve DC bio-impedance measurement accuracy, a multi-purpose chopper is allocated at IA input. Before bio-impedance evaluation, the switch can short the TC inputs to measure the systematic offsets presented at the output of IMP-I and IMP-Q. After calibration phase, the TC inputs are connected to a complex bio-impedance to measure actual voltage value generated by the injected current.

To measure accuracy impedance, an implementation proposes two different modes, a calibration mode and a measurement mode. In the calibration mode, both inputs of the impedance channel are connected to an internal voltage reference and a common mode DC output of (VOUT_DC) can be measured. In the measurement mode, the IA input is connected to external HPF capacitor to measure the bio-impedance RBIO. Based on these two measurements, the bio-impedance can be calculated as follows:

$$R_{BIO} = \frac{V_{OUT} - V_{OUT\_DC}}{I_{AMP} \times GAIN_{CHN}}$$

where, IAMP is the amplitude of injection current and GAINCHN is the impedance channel gain.

According to an exemplary embodiment, two quadrature bio-impedance readout channels are used together with a digitized sinusoidal current generator in order to implement an accurate and wide dynamic range bio-impedance measurement.

According to an exemplary embodiment, to extract both DC and AC bio-impedance components, a non-inverting DC coupled PGA is used, which amplifies the AC bio-impedance while having unity DC gain. This enables a large dynamic range (82dB) bio-impedance readout, which is important as the DC impedance is in general 100 times larger than the AC one. The offset of the quadrature impedance readout circuit may affect the accuracy of the DC bio-impedance calculation. To improve DC bio-impedance measurement accuracy, a multi-purpose chopper is allocated at IA input. Before bio-impedance evaluation, the switch can short the TC inputs to measure the systematic offsets presented at the output of IMP-I and IMP-Q. These offset values are calibrated in the DSP to improve the system accuracy to 97% (THD is less than 3%).

**Claims**

1. A system for impedance measurement comprising:

    a current generator unit (10) configured to generate a differential alternate current signal (11, 12) that is injected to an element (20) that presents a certain electrical impedance (Z);
    electrode sensors configured to provide an analogue sensor signal (S1, S2) sensed from that element (20) when said current signal (11, 12) runs through said element;
    a high-pass filtering unit (30) configured to adapt said analogue sensor signal (S1, S2), so as to reject DC offset, into a filtered analogue signal (IN1, IN2);
    a calibration circuit (40) comprising a switch matrix (43) connected to a chopper (46);
    an analogue impedance readout unit (50) configured to calculate, derived from said filtered signal (IN1, IN2), an impedance value signal (IMP1, IMP2) of the electrical impedance (Z);

an ADC unit (70) configured to digitize the impedance signal (IMP1, IMP2) generated by the analogue impedance readout unit (50); and

a DSP unit (80) configured to receive a digital version of the impedance signal (D1, D2) and to store it in a memory unit;

**characterized in that,** in operation

during a calibration period (PH1), the calibration circuit (40) is configured to activate the chopper (46) and to control the switch matrix (43) in order to disconnect the filtered analog signal (IN1, IN2) from the chopper inputs and to connect a voltage reference signal (VR) to the chopper inputs, such that the system is configured to calculate an offset error of the analogue impedance readout unit (50) and the ADC unit (70), and the DSP unit (80) is configured to store said offset error value (VoffR, Voffl); and

during a measurement period (PH2), the calibration circuit (40) is configured to deactivate the chopper (46) and to control the switch matrix (43) in order to disconnect the voltage reference signal (VR) from the chopper inputs and to connect the filtered analog signal (IN1, IN2) to the chopper inputs, such that the system is configured to measure impedance value signals (IMP1, IMP2) of the electrical impedance (Z), and the DSP unit (80) is configured to calculate an impedance value (R{z},I{z}) by subtracting the offset error value from the measured impedance value.

2. A system for impedance measurement according to claim 1 wherein the analogue impedance readout unit (50) comprises a quadrature impedance readout circuit.

3. A system for impedance measurement according to claim 1 wherein, during the calibration period (PH1), the calibration circuit (40) is configured to activate the chopper (46) by providing a frequency signal (F1) which has the same frequency as the differential alternate current signal (11, 12) generated by the current generator unit (10).

4. A system for impedance measurement according to any of the previous claims, wherein the calculated offset error comprises a DC offset and drift due to process, voltage and temperature variations of the analogue impedance readout unit (50).

5. A system for impedance measurement according to any of the previous claims, wherein the impedance measurement is a bio-impedance measurement.

6. A biopotential signal acquisition system comprising a system for impedance measurement according to any of claims 1 to 5.

**Patentansprüche**

1. System zur Impedanzmessung, umfassend:

eine Stromgeneratoreinheit (10), die konfiguriert ist, um ein differentielles Wechselstromsignal (I1, I2) zu erzeugen, das in ein Element (20) eingespeist wird, das eine bestimmte elektrische Impedanz (Z) aufweist;

Elektrodensensoren, die konfiguriert sind, um ein analoges Sensorsignal (S1, S2) bereitzustellen, das von diesem Element (20) erfasst wird, wenn das Stromsignal (I1, I2) durch das Element läuft;

eine Hochpassfiltereinheit (30), die konfiguriert ist, um das analoge Sensorsignal (S1, S2), um einen DC-Offset abzulehnen, in ein gefiltertes analoges Signal (IN1, IN2) anzupassen;

eine Kalibrierschaltung (40), die eine Schaltmatrix (43) umfasst, die mit einem Chopper (46) verbunden ist;

eine analoge Impedanz-Ausleseeinheit (50), die konfiguriert ist, um, abgeleitet von dem gefilterten Signal (IN1, IN2), ein Impedanzwertsignal (IMP1, IMP2) der elektrischen Impedanz (Z) zu berechnen;

eine ADC-Einheit (70), die konfiguriert ist, um das von der analogen Impedanz-Ausleseeinheit (50) erzeugte Impedanzsignal (IMP1, IMP2) zu digitalisieren; und

eine DSP-Einheit (80), die konfiguriert ist, um eine digitale Version des Impedanzsignals (D1, D2) zu empfangen und in einer Speichereinheit zu speichern;

**dadurch gekennzeichnet, dass** im Betrieb während einer Kalibrierperiode (PH1) die Kalibrierschaltung (40) konfiguriert ist, um den Chopper (46) zu aktivieren und die Schaltmatrix (43) zu steuern, um das gefilterte analoge Signal (IN1, IN2) von den Chopper-Eingängen zu trennen und ein Spannungsreferenzsignal (VR) mit den Chopper-Eingängen zu verbinden, so dass das System konfiguriert ist, um einen Offsetfehler der analogen Impedanz-Ausleseeinheit (50) und der ADC-Einheit (70) zu berechnen, und die DSP-Einheit (80) konfiguriert ist, um den Offsetfehlerwert (VoffR, Voffl) zu speichern; und während einer Messperiode (PH2) die Kalibrierschaltung (40) konfiguriert ist, um den Chopper (46) zu deaktivieren und die Schaltmatrix (43) zu steuern, um das Spannungsreferenzsignal (VR) von den Chopper-Eingängen zu trennen und das gefilterte analoge Signal (IN1, IN2) mit den Chopper-Eingängen zu verbinden, so dass das System konfiguriert ist, um Impedanzwertsignale (IMP1, IMP2) der elektrischen Impedanz (Z) zu messen, und die DSP-Einheit (80) konfiguriert ist, um einen Impedanzwert (R{z}, I{z}) durch Subtraktion des Offsetfehlerwerts von dem gemessenen Impedanzwert zu berechnen.

**2.** System zur Impedanzmessung nach Anspruch 1, wobei die analoge Impedanz-Ausleseeinheit (50) eine Quadratur-Impedanz-Ausleseeinheit umfasst.

**3.** System zur Impedanzmessung nach Anspruch 1, wobei während der Kalibrierperiode (PH1) die Kalibrierschaltung (40) konfiguriert ist, um den Chopper (46) durch Bereitstellen eines Frequenzsignals (F1), das die gleiche Frequenz wie das von der Stromgeneratoreinheit (10) erzeugte differentielle Wechselstrom-signal (I1, I2) aufweist, zu aktivieren.

**4.** System zur Impedanzmessung nach einem der vorstehenden Ansprüche, wobei der berechnete Offsetfehler einen DC-Offset und eine Drift aufgrund von Manufaktur-, Spannungs- und Temperaturschwankungen der analogen Impedanz-Ausleseeinheit (50) umfasst.

**5.** System zur Impedanzmessung nach einem der vorstehenden Ansprüche, wobei die Impedanzmessung eine Bioimpedanzmessung ist.

**6.** Biopotentialsignalerfassungssystem, das ein System zur Impedanzmessung nach einem der Ansprüche 1 bis 5 umfasst.

## Revendications

**1.** Système de mesure d'impédance comprenant :

une unité génératrice de courant (10) configurée pour générer un signal de courant alternatif différentiel (I1, I2) qui est injecté à un élément (20) qui présente une certaine impédance électrique (Z) ;
des capteurs d'électrodes configurés pour fournir un signal de capteur analogique (S1, S2) détecté dans cet élément (20) lorsque ledit signal de courant (I1, I2) s'écoule à travers ledit élément ;
une unité filtrante passe-haut (30) configurée pour adapter ledit signal de capteur analogique (S1, S2), de manière à rejeter un décalage CC, dans un signal analogique filtré (IN1, IN2) ;
un circuit d'étalonnage (40) comprenant une matrice de commutation (43) connectée à un vibreur (46) ;
une unité de lecture d'impédance analogique (50) configurée pour calculer, tiré dudit signal filtré (IN1, IN2), un signal de valeur d'impédance (IMP1, IMP2) de l'impédance électrique (Z) ;
une unité CAN (70) configurée pour numériser le signal d'impédance (IMP1, IMP2) généré par le circuit de lecture d'impédance analogique (50) ; et
une unité DSP (80) configurée pour recevoir une version numérique du signal d'impédance (D1, D2) et la stocker dans une unité de mémoire ;
**caractérisé en ce que,** en service,
au cours d'une période d'étalonnage (PH1), le circuit d'étalonnage (40) est configuré pour activer le vibreur (46) et commander la matrice de commutation (43) afin de déconnecter le signal analogique filtré (IN1, IN2) des entrées du vibreur et connecter un signal de référence de tension (VR) aux entrées du vibreur en sorte que le système soit configuré pour calculer une erreur de décalage de l'unité de lecture d'impédance analogique (50) et de l'unité CAN (70) et que l'unité DSP (80) soit configurée pour stocker ladite valeur d'erreur de décalage (VoffR, VoffI) ; et,
au cours d'une période de mesure (PH2), le circuit d'étalonnage (40) est configuré pour désactiver le vibreur (46) et commander la matrice de commutation (43) afin de déconnecter le signal de référence de tension (VR) des entrées du vibreur et connecter le signal analogique filtré (IN1, IN2) aux entrées du vibreur en sorte que le système soit configuré pour mesurer des signaux de valeurs d'impédance (IMP1, IMP2) de l'impédance électrique (Z) et que l'unité DSP (80) soit configurée pour calculer une valeur d'impédance (R{z}, I{z}) en soustrayant la valeur d'erreur de décalage de la valeur d'impédance mesurée.

**2.** Système de mesure d'impédance selon la revendication 1, dans lequel l'unité de lecture d'impédance analogique (50) comprend un circuit de lecture d'impédance en quadrature.

**3.** Système de mesure d'impédance selon la revendication 1, dans lequel, au cours de la période d'étalonnage (PH1), le circuit d'étalonnage (40) est configuré pour activer le vibreur (46) en fournissant un signal de fréquence (F1) qui a la même fréquence que le signal de courant alternatif différentiel (I1, I2) généré par l'unité génératrice de courant (10).

**4.** Système de mesure d'impédance selon l'une quelconque des revendications précédentes, dans lequel l'erreur de décalage calculée comprend un décalage CC et une dérive due aux variations de manufacture, tension et de température de l'unité de lecture d'impédance analogique (50).

**5.** Système de mesure d'impédance selon l'une quelconque des revendications précédentes, dans lequel la mesure d'impédance est une mesure de bio-impédance.

**EP 2 767 230 B1**

**6.** Système d'acquisition de signal de bio-potentiel comprenant un système de mesure d'impédance selon l'une quelconque des revendications 1 à 5.

**Figure 1**

**Figure 2**

**Figure 3**

**Figure 4**

**Figure 5A**

PH1

40

43

C3
S3

C1
S1

C2 S2

IN1

C5
S5
VR
S6
C6

C4 S4

IN2

C10 46

IN1'

IN2'

IC1

IC2

C11

C12 48

F1 VD

**Figure 5B**

PH1

60

40 50

IN1

IN2

IC1

IC2

IMP11

IMP21

**Figure 6A**

PH2

**Figure 6B**

PH2

**Figure 7A**

**Figure 7B**

**Figure 8**

**Figure 9**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2298164 A

- US 5467090 A

**Non-patent literature cited in the description**

- **CHRISTIAN ENZ.** A CMOS chopper amplifier. *IEEE Journal of Solid-State Circuits,* June 1987, vol. SC-22 (3